(19) Europäisches Patentamt
European Patent Office
Office européen des brevets

(11) Publication number: **0 016 660**
**B1**

(12) **EUROPEAN PATENT SPECIFICATION**

(45) Date of publication of patent specification: **26.10.83**

(21) Application number: **80300942.2**

(22) Date of filing: **26.03.80**

(51) Int. Cl.³: **C 07 D 263/14,**
C 10 L 1/24, C 10 M 1/38
//C07D307/60

(54) **Thio-bis-(hydrocarbon-bisoxazolines) and analogs, process for their preparation and their use as oleaginous additives.**

(30) Priority: **26.03.79 US 23615**

(43) Date of publication of application:
**01.10.80 Bulletin 80/20**

(45) Publication of the grant of the patent:
**26.10.83 Bulletin 83/43**

(84) Designated Contracting States:
**DE FR GB IT**

(56) References cited:
DE - A - 2 512 201
US - A - 3 563 920

(73) Proprietor: **Exxon Research and Engineering Company**
**P.O.Box 390 180 Park Avenue**
**Florham Park New Jersey 07932 (US)**

(72) Inventor: **Brois, Stanley James**
**5318 Brownlee Lane**
**Spring Texas (US)**
Inventor: **Gutierrez, Antonio**
**22 Tar Heels Road**
**Mercerville New Jersey (US)**

(74) Representative: **Bawden, Peter Charles**
**Abingdon Chemical Research Centre P O Box 1**
**Abingdon, OX13 6BB (GB)**

**EP 0 016 660 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Thio-bis-(hydrocarbon-bisoxazolines) and analogs, process for their preparation and their use as oleaginous additives

The present invention concerns oil-soluble thio-bis-(hydrocarbon-bisoxazolines) and their analogs, their method of preparation and the utility of said oxazolines in hydrocarbon fuel and lubricating systems as highly stable anti-corrosion agents and/or sludge dispersants.

During the past decade, ashless sludge dispersants have become increasingly important, primarily in improving the performance of lubricants and gasoline in keeping the engine clean of deposits, and permitting extended crankcase oil drain periods. Most commercial ashless dispersants fall into several general categories. In one category, a poly-amine is linked to a long-chain hydrocarbon polymer, such as polyisobutylene, through a dicarboxylic acid material, such as succinic anhydride, by forming amide or imide linkages, such as described in U.S. Patent 3,172,892.

Unit Kingdom Specification 809,001 teaches corrosion inhibitors comprising a multiple salt complex derived from the reaction product of hydrocarbyl-substituted dicarboxylic acids and hydroxy amines (including 2-amino-2-methyl-1,3-propanediol [AMPD]) and *tris*-hydroxymethylaminomethane (THAM) further complexed with mono- and polycarboxylic acids (see Examples 17—19). United Kingdom Specificatoin 984,409 teaches ashless, amide/imide/ester type lubricant additives prepared by reacting an alkenyl succinic anhydride, said alkenyl group having 30 to 700 carbon atoms, with a hydroxy amine including THAM. German OS 2512201 teaches reacting long-chain hydrocarbon-substituted succinic anhydride with 2,2-disubstituted-2-amino-1-alkanol to produce mon- and bisoxazoline products. Thio-bis-(alkyl lactone oxazolines), useful as oleaginous additives, and chlorine-containing thio-bis-adducts of sulfur chlorides with octenyl succinic anhydride are disclosed in U.S.P. 4,062,786.

It has now been discovered that the dehydrochlorinated thio-bis-adducts or acylating agents obtained by the reaction of sulfur halides with hydrocarbyl-substituted dicarboxylic anhydrides or acids or esters can be further reacted with 2,2-disubstituted-2-amino 1-alkanols to form oxazolines.

The present invention therefore provides a proces for preparing thio-bis-(hydrocarbon-bisoxazolines) comprising the step of reacting one molar proportion of an acylating reagent (thio bis adduct) of the formula

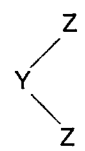

where Y is —S— or —S—S— and Z is the residue of a product obtained by reacting maleic or succinic acid or an ester or anhydride thereof with an olefine polymer having a number average molecular weight in the range 500 to 140,000, with from 1 to 4 molar proportions of a 2,2-disubstituted -2-amino-1-alkanol containing a total of 4 to 8 carbon atoms represented by the formula:

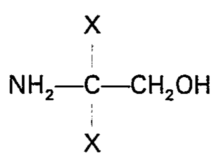

wherein X is an alkyl or hydroxyalkyl group, with at least one of the X substituents being a hydroxy alkyl group of the structure $—(CH_2)_nOH$, wherein n is 1 to 3, until the reaction product shows maximal absorption for oxazoline as measured by infrared analysis.

The reaction of the thio-bis-adducts or acylating agents is carried out using 1 to 4, preferably 2 to 4 molar equivalents of the alkanol per molar equivalent of said adduct. The most preferred proportions comprise 4 moles of the alkanol per mole of said acylating agent thereby forming a thio-bis-(hydrocarbon-bis-oxazoline) having about 4 oxazoline groups per molecule. The oxazolines are highly stable additives with outstanding varnish inhibition. A preferred member of this novel class of additives can be represented by the formula:

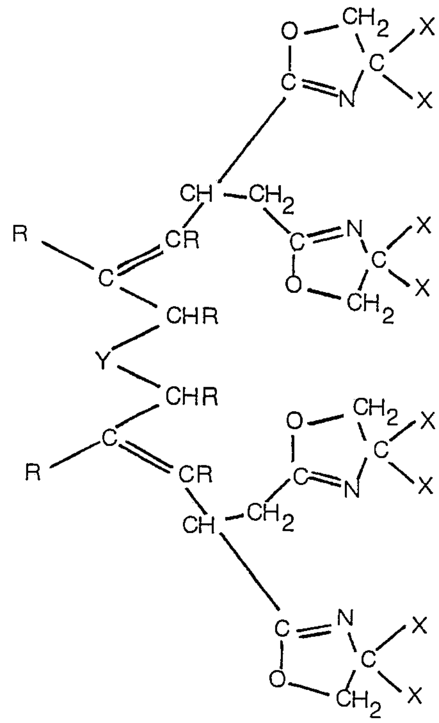

wherein R is hydrogen or is derived from an olefine polymer of molecular weight 500 to 140,000, X is an alkyl or hydroxyalkyl group and at least one of the X substituents being a hydroxy alkyl group of the structure —$(CH_2)_nOH$ where n is 1 to 3, and Y is —S— or —S—S—.

Preferred herein is thio-bis-(polyisobutenyl-bis-oxazoline) of number average molecular weight ranging from about 400 to 100,000 prepared by the reaction of thio-bis (acylating reagent), e.g. thio-bis-(polyisobutenyl) succinic anhydride, with tetramolar proportions of *tris*-(hydroxymethyl) amino-methane (hereinafter designated also as THAM). The reaction is carried out at a temperature from 100 to 240°C., preferably 150—180°C, until about six moles of $H_2O$ per mole of the thio reactant are removed from the reaction.

The novel compounds described above as effective detergents in lubricating oil compositions are also useful as detergents in fuel composition, such as burner fuel compositions, and motor fuel compositions, for example, in gasolines and in diesel fuels.

The preparation of the acylating agents involves the sulfur halide coupling or bis-sulfenyl halide-induced coupling. The olefin diacid material is readily obtained via the reaction of an olefin or a chlorinated olefin with maleic or succinic acids or anhydrides or esters thereof. The dicarboxylic acid material formed via the Ene reaction of an olefin with maleic anhydride can be illustrated as an alkenyl-substituted anhydride which may contain a single alkenyl radical or a mixture of alkenyl radicals variously bonded to the cyclic succinic anhydride group, and is understood to comprise such structures as:

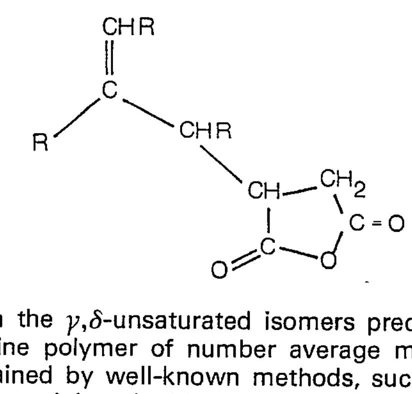

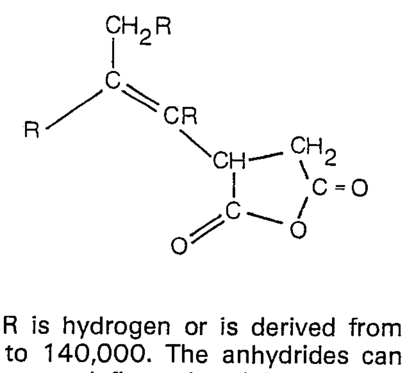

with the $\gamma,\delta$-unsaturated isomers predominating and wherein R is hydrogen or is derived from an olefine polymer of number average molecular weight of 500 to 140,000. The anhydrides can be obtained by well-known methods, such as the reaction between an olefin and maleic anhydride or halosuccinic anhydride or succinic ester. In branched olefins, particularly branched polyolefins, R may be hydrogen, methyl or a long-chain hydrocarbyl group. However, the exact structure may not always be ascertained and the various R groups cannot always be precisely defined in the Ene products from polyolefins and maleic anhydride.

Suitable olefins include polymers of butene, isobutene, pentene, decene, dodecene, tetradecene,

hexadecene, octadecene, eicosene, and polymers of propylene, butene, isobutene, pentene, decene and the like. Bridging of these products with $YCl_2$ also afford useful precursors. Preferred olefin polymers for reaction with the unsaturated dicarboxylic acids are polymers comprising a major molar amount of $C_2$—$C_5$ monoolefin, e.g., ethylene, propylene, butylene, isobutylene and pentene. The polymers can be homopolymers, such as polyisobutylene, as well as copolymers of two or more of such olefins such as copolymers of ethylene and propylene, butylene and isobutylene, propylene and isobutylene, etc.

The olefin polymers will preferably have number average molecular weight ($M_n$) within the range of between 700 and 5,000 with approximately one terminal double bond per polymer chain. An especially valuable starting material for a highly potent dispersant additive are polyalkenes, e.g. polypropylene and polyisobutylene, having about 90 carbons.

The bridging or coupling of the precursor acylating agents can conveniently be achieved by the addition of sulfur halides or bis-sulfenyl halides or alkyl sulfenate/HCl reagent to unsaturated diacid anhydrides.

The preferred method to bridged acylating agents involves the reaction of sulfur halides or bis-sulfenyl halides in the temperature range of −60°C to 100°C, optimally from 10°C to 50°C. If desired, solvents comprising hydrocarbons, such as pentane, hexane heptane, cyclohexane, mineral oil; halocarbons such as methylene chloride, chlororform, carbon tetrachloride, aromatics such as toluene, chlorobenzenes, xylene; ethers, such as diethyl ether and tetrahydrofuran (THF); and, acids such as acetic, propionic and trifluoroacetic acid, can be used in favorably controlling viscosity and reaction temperature. Usually, the sulfur halide is added dropwise to an unsaturated diacid anhydride, preferably diluted in an inert diluent. With reactive diluents and unsaturates such as polyisobutylene, sufficient sulfur halide must be added to effect complete bridging to the olefin diacid anhydride reactants.

When the addition of one mole of sulfenyl halide to 2 moles of alkene dioic acid anhydride is conducted at low temperatures, e.g. −60°C to 20°C, a discrete $YCl_2$-anhydride adduct forms which upon dehydrohalogenation gives a thio-bis-acylating agent. The preferred material of the present invention is therefore a thio-ether prepared by reacting the reaction product of an olefine polymer having a number average molecular weight inthe range of 500 to 140,000 with succinic anhydride, acid or ester, with sulphur monochloride or sulphur dichloride to form a chlorine-containing adduct, dehydrochlorinating the adduct by heating to form a mono- or dithio-bis-(hydro-carbyl succinic anhydride or ester) and then reacting one molar proportion of said mono- or dithio-bis-product with from 1 to 4 molar proportions of a 2,2-disubstituted-2-amino-1-alkanol containing a total of 4 to 8 carbon atoms represented by the formula:

$$\begin{array}{c} X \\ | \\ NH_2\text{—}C\text{—}CH_2OH \\ | \\ X \end{array}$$

wherein X is an alkyl or hydroxyalkyl group, with at least one of the X substituents being a hydroxy alkyl group of the structure $(CH_2)_nOH$, wherein n is 1 to 3 until the reaction product shows maximal absorption for oxazoline as measured by infrared analysis.

Increasing the bridging temperature above about 50°C., and branching in the hydrocarbyl portion of the alkene dioic anhydride tend to accelerate the elimination of HCl from $YCl_2$-alkene dioic anhydride adduct. Since unsaturated bridging products can be further sulfenylated with $YCl_2$ reagent (readdition), it becomes necessary in some cases, to modify the theoretical 2 : 1 stoichiometry to effect complete bridging. Accordingly, at higher temperatures, i.e. from 50°—100°C., ratios in the range of 1.5 : 1 to 1 : 1 may be required to realize higher conversions to bridged structures due to re-addition reactions, and the partial thermal decomposition of the sulfur halide reactant at elevated temepratures. While more sulfur halide reagent become necessary to achieve coupling, the additional sulfur incorporated into the dispersant precursor (and occasionally the diluent) tends to endow the resulting thio-ether products with enhanced oxidative stability.

Examples of the amino alcohols of the formula:

$$\begin{array}{c} X \\ | \\ NH_2\text{—}C\text{—}CH_2OH \\ | \\ X \end{array}$$

include 2-amino-2-methyl-1,3-propanediol, 2-amino-2-(hydroxy-methyl)-1,3-propanediol (also known as *tris*-hydroxy aminomethane or THAM), 2-amino-2-ethyl-1,3-propanediol, etc. Because of its effectiveness availability, and cost, the THAM is particularly preferred.

By sharp contrast, it has been found that other amino alcohols such as ethanolamine, propanolamine and butanolamine which lack, 2,2-disubstitution, do not afford oxazoline products.

The formation of the novel oxazoline materials in substantial yield, can be effected by adding from one to four moles of the aforesaid 2,2-disubstituted-2-amino-1-alkanol per mole of the thio-bis-(acylating reagent) with or without an inert diluent, and heating the mixture at 100—240°C., preferably 170—220°C. until reaction is complete by infrared analysis of the product showing maximal absorption for oxazoline and/or until evolution of water ceases.

Although not necessary, the presence of small amounts such as .01 to 2 wt.%, preferably 0.1 to 1 wt.%, based on the weight of the reactants, of a metal salt can be used in the reaction mixture as a catalyst. The metal catalyst can later be removed by filtration or by washing a hydrocarbon solution of the product with a lower alcohol, such as methanol, ethanol, isopropanol, etc., or an alcohol/water solution.

Alternatively, the metal salt can be left in the reaction mixture, as it appears to become stably dispersed, or dissolved, in the reaction product,and depending on the metal, it may even contribute performance benefits to the oil or gasoline. This is believed to occur with the use of zinc catalysts in lubricants.

Inert solvents which may be used in the above reaction include hydrocarbon oils, e.g. mineral lubricating oil, kerosene, neutral mineral oils, xylene, halogenated hydrocarbons, e.g., carbon tetrachloride, dichlorobenzene, tetrahydrofuran, etc.

Metal salts that may be used as catalysts in the invention include carboxylic acid salts of Zn, Co, Mn and Fe. Metal catalysts derived from strong acids (HCl, sulfonic acid, $H_2SO_4$, $HNO_3$, etc.) and bases, tend to diminish the yield of the axazoline products and instead favor imide or ester formation. For this reason, these strong acid catalysts or basic catalysts are not preferred and usually will be avoided. The carboxylic acids used to prepare the desired catalysts, include $C_1$ to $C_{18}$, e.g., $C_1$ to $C_8$ acids, such as the saturated or unsaturated mono- and dicarboxylic aliphatic hydrocarbon acids, particularly fatty acids. Specific examples of such desired carboxylic acid salts include zinc acetate, zinc formate, zinc propionate, zinc stearate, manganese(ous) acetate, iron tartrate, cobalt(ous) acetate, etc. Completion of the oxazoline reaction can be readily ascertained by using periodic infrared spectral analysis for maximal oxazoline formation (C-N absorption band at 6.0 microns) and/or by the cessation of water evolution.

In another embodiment of the invention the thio-bis-(acylating reagent) is treated with less than a stoichiometric quantity, i.e. less than four molar equivalents e.g. 1 to 3, preferably 2 to 3, molar equivalents of THAM, to provide oxazoline-ester dispersants which are useful in themselves or can be converted to other classes of novel dispersants by treatment with polyamines to form oxazoline imide/amide products or reaction with polyols, such as pentaerythritol, preferably using ratios of 1 molar equivalent of said oxazoline-ester dispersant per 1 to 2 molar equivalents of pentaerythritol, to form novel oxazoline polyol dispersants and if desired finally reacted with alkylene oxides to form alkoxylated derivatives thereof having wide utility as additives.

It is to be understood that the oxazoline products of the invention can be both molybdated with molybdenum to enhance their lubricity activity and borated with boron to enhance the additives' anti-corrosion and/or varnish inhibition activities.

The oil-soluble oxazoline reaction products of the invention can be incorporated in a wide variety of oleaginous compositoins. They can be used in lubricating oil compositions, such as automotive crankcase lubricating oils, automatic transmission fluids, etc., in concentrations generally within the range of 0.01 to 20 wt.%, e.g. 0.1 to 10 weight percent, preferably 0.3 to 3.0 weight percent, of the total composition. The lubricants to which the oxazoline products can be added include not only hydrocarbon oils derived from petroleum but also include synthetic lubricating oils such as polyethylene oils; alkyl esters of dicarboxylic acid; complex esters of dicarboxylic acid, polyglycol and alcohol; alkyl esters of carbonic or phosphoric acids; polysilicones; fluorohydrocarbon oils; mixtures of mineral lubricating oil and synthetic oils in any proportion, etc.

When the products of this invention are used as multifunctional additives having detergent and antirust properties in petroleum fuels, such as gasoline, kerosene, diesel fuels, No. 2 fuel oil and other middle distillates, a concentration of the additive in the fuel in the range of 0.001 to 0.5 weight percent, based on the weight of the total composition, will usually be employed.

When used as an antifoulant in oil streams in refinery operations to prevent fouling of process equipment such as heat exchangers or in turbine oils, about 0.001 to 2 wt.% will generally be used.

The additive may be conveniently dispensed as a concentrate comprising 20 to 90 parts, preferably 30 to 60, parts by weight, of the additive dissolved in a mineral lubricating oil.

Example 1

Dithio-bis[polyisobutenyl-bis-(5,5,-bis-methylol-2-oxazoline)]

Two-hundred grams (*ca* 0.154 mole) of a polyisobutenyl succinic anhydride (prepared via the reaction of polyisobutene and maleic anhydride) having a ($\bar{M}_n$) of 1300 and a Saponification Number of 72, were diluted with 100 ml of methylene chloride and stirred at room temperature under a nitogen blanket. Then, 10.4 g (*ca* 0.077 mole) of $S_2Cl_2$ were added dropwise for a period of half an hour. The reaction mixture was stirred at room temperature for about ten hours.

One-half of this product was evaporated and the residue was sparged with nitrogen at 150°C. for

four hours. The resulting dithio-bis-(polyisobutyenyl succinic anhydride) adduct analyzed for 2.08 wt.% S and 0.15 wt.% Cl.

About 21 g (*ca* 0.007 moles) of said adduct were diluted with an equal weight of mineral oil (Solvent 150 Neutral) and heated to 120°C. Then 0.1 g of zinc acetate and 3.4 g (0.028 mole) of THAM were added. The reaction mixture was heated to 180°C. for 2 hours while sparging with nitrogen and filtered. The oil solution analyzed for 0.9 wt.% nitrogen. The infrared analysis confirmed the presence of the above-identified tetraoxazoline product.

Example 2

Dithio-bis-[polyisobutyenyl-bis-(5,5-bis-methylol-2-oxazoline]

30 g (*ca* 0.014 mole) of a dithio-bis-(polyisobutenyl succinic anhydride) adduct derived from a polyisobutenyl succinic anhydride of ($\bar{M}_n$) of 990 and a Sap. No. 107 and $S_2Cl_2$ were diluted with 32 g of mineral oil (Solvent 150 Neutral) and heated to 120°C. Then 0.1 g of zinc diacetate and 6.9 g (*ca* 0.057 moles) of THAM were added. The reaction mixture was heated to 180°C. for two hours with nitrogen sparging and then filtered. The oil solution of the oxazoline product analyzed for 1.07 wt.% nitrogen.

Example 3

Thio-bis-[polyisobutenyl-bis-(5,5-bis-methylol-2-oxazoline)]

Five-hundred grams (*ca* 0.385 moles) of polyisobutenylsuccinic anhydride having a ($\bar{M}_n$) of 775 and a Sap No. of 84 were dissolved in 60 ml of methylene chloride and cooled to 0°C. While stirring at 0°C. under a nitrogen atmosphere, 19.8 g (*ca* 0.192 mole) of $SCl_2$ were added dropwise for a period of half hour. The reaction mixture was allowed to warm up to room temperature and stirred for about ten hours.

One-half of this adduct product was dehydrohalogenated by rotoevaporation under high vacuum for 6 hours at about 100°C.

The adduct analyzed for 1.34 wt.% S and 0.70 wt.% Cl. The infrared analysis was consistent with that of a thio-bis (polyisobutenylsuccinic anhydride) adduct.

About 80 g (*ca* 0.03 moles) of said adduct were diluted with an equal amount of mineral oil (solvent 150 Neutral) and heated to 130°C. Then 0.1 g of zinc acetate dihydrate were added, followed by the addition of 14.5 g (*ca* 0.12 moles) of THAM. The reaction mixture was heated slowly to 180°C. and kept at this temperature for 2 hours while sparged with nitrogen. The oil solution was filtered and the filtrate analyzed for 1.27 wt.% nitrogen. An infrared spectrum of this product confirmed the presence of the above-identified tetraoxazoline.

Example 4

Dithio-bis-[polyisobutenyl-bis-(5,5-bis-methylol-2-oxazoline)]

Five hundred grams (*ca* 0.385 moles) of the polyisobutylene succinic anhydride used in Example 3 were dissolved in 60 ml of methylene chloride and cooled to 0°C. While stirring at 0°C. under a nitrogen blanket, 26 g (ca 0.192 moles) of sulfur monochloride were added dropwise for a period of half hour. The reaction mixture was allowed to warm up to room temperature and stirred for about ten hours.

One-half of this product was rotoevaporated under high vacuum at 100°C. for 6 hours. The resulting adduct analyzed for 2.43 wt.% S and 0.65 wt.% Cl.

Approximately 50 g (*ca* 0.02 moles) of the dithio-bis-(polyisobutenyl succinic anhydride) adduct were mixed with 0.1 g of zinc diacetate and diluted with an equal amount of mineral oil (Solvent 150 Neutral). The oil solution was heated to 120°C. and 9.7 g (*ca* 0.08 mole) of THAM were added. The reaction temperature was slowly raised to 180°C. and kept at this temperature for two hours. At the end of the second hour the oil solution was filtered. The filtrate analyzed for 1.14 wt.% nitrogen. The infrared analysis featured strong absorption band at 6.0—6.05 microns confirming the presence of an oxazoline reaction product.

Example 5

Reaction product of 1 molar equivalent of thio-bis-adduct with 2 molar equivalents of THAM

About 108 g (0.04 mole) of the thio-bis-(polyisobutenylsuccinic anhydride) adduct of Example 3 was reacted with 9.7 g (0.08 mole) of THAM under the same conditions as described in Example 3. The product analyzed for 0.63 wt.% nitrogen and 1.36% sulfur.

Example 6

Reaction product of 1 molar equivalent of thio-bis-adduct with 3 molar equivalents of THAM.

About 108 g (0.04 mole) of the adduct of Example 3 was reacted with 14.5 g (0.12 mole) of THAM under the same condition described in Example 3. The product analyzed for 0.94 wt.% nitrogen and 1.18% sulfur.

Example 7

Reaction product of 1 molar equivalent of thio-bis-adduct with 1 molar equivalent of THAM and then reacted with 2 molar equivalents pentaerythritol.

About 108 g (0.04 mole) of the adduct of Example 3 was reacted with 4.8 (0.04 mole) of THAM under the same conditions described in Example 3. The product analyzed for 0.41 wt.% nitrogen and 1.36% sulfur.

About 56 g (0.02 mole) of this product dissolved in 54 g of Solvent 150 Neutral Base oil, was reacted with 5.4 g (0.04 mole) of pentaerythritol (PE) by heating at 210°C for 2 hours while passing nitrogen through the mixture. The reaction product was then purged with nitrogen for 1/2 hour, filtered and recovered. The product analyzed for 0.43 wt.% nitrogen and 1.23 wt.% sulfur; some unreacted PE was recovered.

Example 8

One molar-equivalent of the 1 : 2 product of Example 5 was reacted with 2 molar equivalents of PE using the procedure of Example 7; the product contained 0.73 wt.% nitrogen and 1.28 wt.% sulfur.

Example 9

Sludge Inhibition Bench (SIB) Test

The product of Examples 1, 3 and 4 to 8 and two other dispersant additives were subjected to a Sludge Inhibition Bench (SIB) Test which has been found after a large number of elevations, to be an excellent test for assessing the dispersing power of lubricating oil dispersant additives.

The medium chosen for the Sludge Inhibition Bench Test was a used crankcase mineral lubricating oil composition having an original viscosity of about 325 SUS at 100°F. that had been used in a taxicab that was driven generally for short trips only, thereby causing a buildup of a high concentration of sludge precursors. The oil that was used contained only a refined base mineral lubricating oil, a viscosity index improver, a pour point depressant and zinc dialkyldithiophosphate antiwear additive. The oil contained no sludge dispersants. A quantity of such used oil was acquired by draining and refilling the taxicab crankcase at 1000—2000 mile intervals.

The Sludge Inhibition Bench Test is conducted in the following manner. The aforesaid used crankcase oil, which is milky brown in color, is freed of sludge by centrifuging for 1/2 hour at about 39,000 gravities (gs.). The resulting clear bright red supernatant oil is then decanted from the insoluble sludge particles thereby separated out. However, the supernatant oil still contains oil-soluble sludge precursors which on heating under the conditions employed by this test will tend to form additional oil-insoluble deposits of sludge. The sludge inhibiting properties of the additives being tested are determined by adding to portions of the superntant used oil, a small amount, such as 0.5, 1.0 to 1.5 weight percent, on an active ingredient basis, of the particular additive being tested. Ten grams of each blend being tested in placed in a stainless steel centrifuge tube and is heated at 280°F (137.5°C) for 16 hours in the presence of air. Following the heating, the tube containing the oil being tested is cooled and then centrifuged for 30 minutes at about 39,000 gs. Any deposits of new sludge that form in this step are separated from the oil by decanting the supernatant oil and then carefully washing the sludge deposits with 15 ml. of pentane to remove all remaining oil from the sludge. Then the weight of the new solid sludge that has been formed in the test, in milligrams, is determined by drying the residue and weighing it. The results are reported as milligrams of sludge per 10 grams of oil, thus measuring differences as small as 1 part per 10,000. The less new sludge formed the more effective is the additive as a sludge dispersant. In other words, if the additive is effective, it will hold at least a portion of the new sludge that forms on heating and oxidation, stably suspended in the oil so it does not precipitate down during the centrifuging.

VARNISH INHIBITION BENCH (VIB) TEST

In this (VIB) test each test sample consisted of 10 grams of lubricating oil containing 0.07 of a gram of the additive concentrate (50% active) which results in a total of 0.35 wt.% additive present in the test sample. The test oil to which the additive is admixed was 9.93 grams of a commercial lubricating oil obtained from a taxi after 2,000 miles (3,200 kilometres) of driving with said lubricating oil. Each ten gram sample was heat soaked overnight at about 140°C. and thereafter centrifuged to remove the sludge. The supernatant fluid of each sample was subjected to heat cycling from about 150°C. to room temperature over a period of 3.5 hours at a frequency of about 2 cycles per minute. During the heating phase, the gas containing a mixture of about 0.7 volume percent $SO_2$, 1.4 volume percent NO and balance air was bubbled through the test samples and during the cooling phase water vapor was bubbled through the test samples. At the end of the test period, which testing cycle can be repeated as necessary to determine the inhibiting effect of any additive, the wall surfaces of the test flasks in which the samples were contained are visually evaluated. Flasks in which the samples were contained are visually evaluated as to the varnish inhibition. The amount of varnish imposed on the walls is rated at values of from 1 to 7 with the higher number being the greater amount of varnish. It has been found that this test correlates with the varnish results obtained as a consequence of carrying out an MS—VC engine test.

Using the above-described tests, the dispersant action of the oxazoline additives of the present invention were compared with the dispersing power of one commercial dispersant referred to as PIBSA/TEPA. The PIBSA/TEPA was prepared by reaction of 1 mole of tetraethylene pentamine with

about 2 moles of polyisobutenyl succinic anhydride obtained from polyisobutylene of about 1000 number average molecular weight. The PIBSA/TEPA dispersants each used the form of an additive concentrate containing about 50 wt.% PIBSA/TEPA in 50 wt.% mineral lubricating oil.

In addition, the oxazoline product of the present invention was also compared with polyisobutenylsuccinic anhydride-bisoxazoline material prepared in accordance with the teachings of DOS 2512201 in the Sludge Inhibition Bench Test. The bisoxazoline designated PIBSA/bis-oxazoline dispersant was prepared via the reaction of 2 molar proportions of tris-(hydroxymethyl) aminomethane with polyisobutenylsuccinic anhydride according to the procedure specified in this patent specification. The test results are given in Table I below.

TABLE I

| Additive | | wt.%N | Mg Sludge/ 10g Oil at 0.5 wt.% | VIB Test Rating |
|---|---|---|---|---|
| Blank | | — | 20.2 | 11 |
| PIBSA/TEPA | | 1.5—1.6 | 9.8 | 7 |
| PIBSA/bis-oxazoline | | 1.0 | 11.9 | 7 |
| Example 1 | | 0.9 | 2.0 | 5 |
| ,, | 3 | 1.27 | 10.4 | 6 |
| ,, | 4 | 1.14 | 8.8 | 7 |
| ,, | 5 | 0.63 | 13.8 | 6 |
| ,, | 6 | 0.94 | 10.4 | 5 |
| ,, | 7 | 0.43 | 14.8 | 6.5 |
| ,, | 8 | 0.73 | 13.2 | 6 |

The data of Table I illustrates the superior dispersancy and/or varnish-inhibition activity of the additive products of the invention when prepared with commercial dispersants known as PIBSA/TEPA and PIBSA/bis-oxazoline.

Example 10

The sulfur-bridged products of the present invention and a commercial dispersant additive diluted in mineral oil were evaluated by thermogravimetric analysis (TGA) for evidence of thermal stability under oxidative conditions provided by air flow across each sample heated linearly from about 50°C. to 450°C. at a rate of 20°/min. Each sample of 5—8 mg (as a Solvent 150 N mineral oil solution containing 50 wt.% of an additive) in a platinum planchette was continuously weighed and recorded as the temperature was programmed upwardly at a linear rate to provide a record of sample weight loss versus temperature. The results are found in Table II.

Table II

| Product Additive Tested | Temperature at which the indicated percentage weight loss occurred 10 Wt.% °C. | 50 Wt.% °C. | 70 Wt.% °C. | 90 Wt.% °C. |
|---|---|---|---|---|
| Solventy 150 Mineral Oil | 230 | 283 | 295 | 310 |
| Ex. 1 | 275 | 365 | 410 | 440 |
| Ex. 3 | 270 | 370 | 410 | 440 |
| Ex. 4 | 275 | 375 | 410 | 440 |
| PIBSA/bis-oxazoline | 235 | 330 | 400 | 430 |

The TGA data shown in Table II reveal that the compositions of the present invention are significantly more stable towards heat and oxidation than the reference commercial oxazoline dispersant. In addition, the TGA data show that the tetraoxazolines of the present invention tend to stabilize the base oil, e.g. S-150N base stock oil, towards thermal oxidative degradation. Thus, the novel structural features built into the present dispersants endow these additives with enhanced thermal stability as well as the ability to inhibit oxidation of the base stock oil. It is believed that these inhibitor properties can be related in part to the presence of sulfide functionality present in the additive molecules of the present invention.

## Claims

1. A process for preparing thio-bis-(hydrocarbon-bisoxazolines) comprising the step of reacting one molar proportion of an acylating reagent of the formula

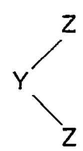

where Y is —S— or —S—S— and Z is the residue of a product obtained by reacting maleic or succinic acid or an ester or, anhydride thereof with an olefine polymer having a number average molecular weight in the range 500 to 140,000 with from 1 to 4 molar proportions of a 2,2-disubstituted-2-amino-1-alkanol containing a total of 4 to 8 carbon atoms represented by the formula:

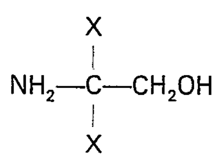

wherein X is an alkyl or hydroxyalkyl group, with at least one of the X substituents being a hydroxy alkyl group of the structure $-(CH_2)_nOH$, wherein n is 1 to 3, until the reaction product shows maximal absorption for oxazoline as measured by infrared analysis.

2. The process according to claim 1 wherein said acylating reagent is thio-bis-(polyisobutenylsuccinic anhydride) and said alkanol is tris (hydroxymethyl) amino-methane.

3. The process according to claims 1 to 2 wherein 4 molar proportions of said alkanol are reacted with one molar proportion of said acylating agent.

4. The process according to claims 1 or 2, wherein 2 to 3 molar proportions of said alkanol are reacted with 1 molar proportion of said acylating agent and said reaction product is then reacted with polyamines and/or polyols in an amount sufficient to react with the remaining carboxyl groups of said acylating agent.

5. The process according to claim 4 wherein said alkanol is tris (hydroxymethyl) amino-methane and said polyol pentaerythritol.

6. A thio-bis-(hydrocarbon-bisoxazoline) prepared by reacting the reaction product of an olefine polymer having a number average molecular weight in the range of 500 to 140000 with succinic anhydride, acid or ester with sulphur monochloride or sulphur dichloride to form a chlorine-containing adduct, dehydrochlorinating the adduct by heating to form a mono- or dithio-bis-(hydro-carbyl succinic anhydride or ester) and then reacting one molar proportion of said mono- or dithio-bis-product with from 1 to 4 molar proportions of a 2,2-disubstituted-2-amino-1-alkanol containing a total of 4 to 8 carbon atoms represented by the formula:

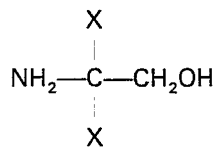

wherein X is an alkyl or hydroxyalkyl group, with at least one of the X substituents being a hydroxy alkyl group of the structure $(CH_2)_nOH$, wherein n is 1 to 3 until the reaction product shows maximal absorption for oxazoline as measured by infrared analysis.

7. A thio-bis-(hydrocarbon-bisoxazoline) according to claim 6 wherein said alkanol is tris (hydroxymethyl) amino-methane.

8. A thio-bis-(hydrocarbon-bisoxazoline) according to claim 6 or 7 wherein 4 molar proportions of said alkanol are reacted with one molar proportion of said mono- or dithio-bis product.

9. A thio-bis-(hydrocarbon-bisoxazoline) according to claims 6—8 wherein said olefine polymer is polyisobutylene.

10. A thio-bis-(hydrocarbon-bisoxazoline according to Claims 6 or 7 wherein said alkanol is used in an amount ranging from 1 to 3 molar proportions and said reaction product is thereafter reacted with polyamines and/or polyols in an amount sufficient to react with the remaining carboxyl groups of said acylating reagent.

11. A thio-bis-(hydrocarbon-bisoxazoline) according to claim 6 represented by the formula:

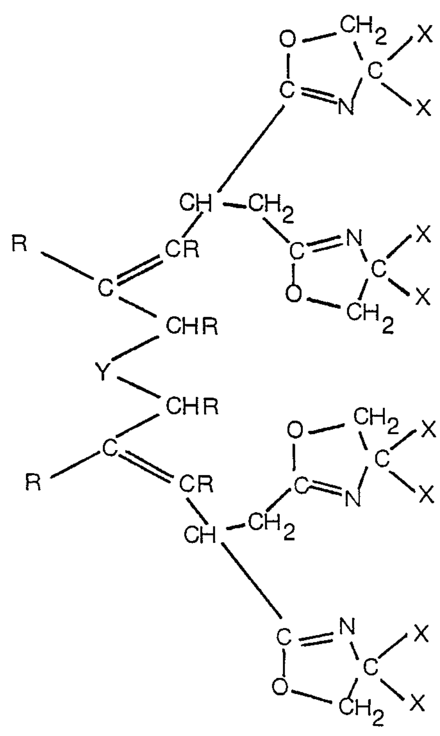

wherein R is hydrogen or is derived from an olefine polymer of number average molecular weight from 500 to 140,000, X is an alkyl or hydroxy alkyl group and at least one of the X substituents being a hydroxy alkyl group of the structure $-(CH_2)_nOH$ where n is 1 to 3, and Y is —S— or —S—S—,

12. A compound according to claim 11 wherein both of said X substituents are a hydroxy alkyl group of the structure $-(CH_2)_nOH$ and n is 1.

13. Dithio-bis-(polyisobutenyl-bis-(5,5,-bis-methylol-2-oxazoline)) according to claim 11.

14. Thio-bis-(polyisobutyenyl-bis-(5,5,-bis-methyol-2-oxazoline)) according to claim 11.

15. A compositoin comprising an oleaginous material of the class consisting of fuels and lubricants and at least a corrosion-inhibiting amount of a compound according to claim 11.

16. A composition according to claim 15 wherein said oleaginous material is a lubricating mineral oil containing 0.01 to 20 wt.% of said compound.

17. A composition according to claim 15 wherein said oleaginous material is a lubricating mineral oil containing from 20—90 wt.% of said compound.

18. A composition according to claim 15 wherein said oleaginous material is gasoline.

**Revendications**

1. Procédé de préparation de thio-bis-(hydrocarbyl-bis-oxazolines), comprenant l'étape de réaction d'une proportion molaire d'un réactif acylant de formule.

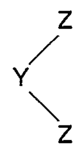

dans laquelle Y représente —S— ou —S—S— et Z est le résidu d'un produit obtenu par réaction d'acide maléique ou succinique ou d'un ester ou anhydride de cet acide avec un polymère oléfinique dont le poids moléculaire a une moyenne en nombre comprise dans la plage de 500 à 140 000, avec 1 à 4 proportions molaires d'un 2-amino-1-alcanol disubstitué en position 2 contenant au total 4 à 8 atomes de carbone représenté par la formule:

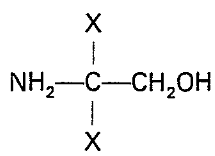

dans laquelle X est un groupe alkyle ou hydroxalkyle, au moins l'un des substituants X étant un groupe hydroxyalkyle de formule —$(CH_2)_n$OH dans laquelle n a une valeur de 1 à 3, jusqu'à ce que le produit réactionnel montre l'absorption maximale pour l'oxazoline, telle que mesurée par l'analyse infrarouge.

2. Procédé suivant la revendication 1, dans lequel ledit réactif acylant est le thio-bis-(anhydride polyisobuténylsuccinique) et ledit alcanol est le tris-(hydroxyméthyl)aminométhane.

3. Procédé suivant la revendication 1 ou 2, dans lequel 4 proportions molaires dudit alcanol sont amenées à réagir avec une proportion molaire dudit agent acylant.

4. Procédé suivant les revendications 1 ou 2, dans lequel 2 à 3 proportions molaires dudit alcanol sont amenées à réagir avec une proportion molaire dudit agent acylant et ledit produit réactionnel est ensuite amené à réagir avec des polyamines et/ou des polyols en une quantité suffisante pour réagir avec les groupes carboxyle restants dudit agent acylant.

5. Procédé suivant la revendication 4, dans lequel ledit alcanol est le tris-(hydroxyméthyl)aminométhane et ledit polyol est le pentaérythritol.

6. Une thio-bis-(hydrocarbyl-bis-oxazoline) préparée par réaction du produit réactionnel d'un polymère oléfinique ayant une moyenne en nombre du poids moléculaire dans la plage de 500 à 140 000 et de l'anhydride, de l'acide ou d'un ester succinique, avec le monochlorure de soufre ou le dichlorure de soufre pour former un produit d'addition contenant du chlore, déshydrochloration du produit d'addition par chauffage pour former un mono- ou dithio-bis-(anhydride ou ester hydrocarbylsuccinique), puis réaction d'une proportion molaire dudit produit mono- ou dithio-bis avec 1 à 4 proportions molaires d'un 2-amino-1-alcanol disubstitué en position 2 contenant au total 4 à 8 atomes de carbone, représenté par la formule:

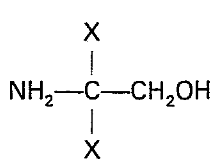

dans laquelle X est un groupe alkyle ou hydroxyalkyle, l'un au moins des substituants X étant un groupe hydroxyalkyle de formule $(CH_2)_n$OH, dans laquelle n a une valeur de 1 à 3 jusqu'à ce que le produit réactionnel présente une absorption maximale pour l'oxazoline telle que mesurée par l'analyse infrarouge.

7. Une thio-bis-(hydrocarbyl-bis-oxazoline) suivant la revendication 6, dans lequel ledit alcanol est le tris-(hydroxyméthyl)aminométhane.

8. Une thio-bis-(hydrocarbyl-bis-oxazoline) suivant la revendication 6 ou 7, dans laquelle 4

proportions molaires dudit alcanol sont amenées à réagir avec un proportion molaire dudit produit mono- ou dithio-bis.

9. Une thio-bis-(hydrocarbyl-bis-oxazoline) suivant les revendications 6—8, dans laquelle ledit polymère oléfinique est le polyisobutylène.

10. Une thio-bis-(hydrocarbyl-bis-oxazoline) suivant les revendications 6 ou 7, dans laquelle ledit alcanol est utilisé en une quantité allant de 1 à 3 proportions molaires et ledit produit réactionnel est ensuite amené à réagir aved des polyamines et/ou des polyols en une quantité suffisante pour réagir avec les groupes carboxyle restants dudit réactif acylant.

11. Une thio-bis-(hydrocarbyl-bis-oxazoline) suivant la revendication 10, représentée par la formule:

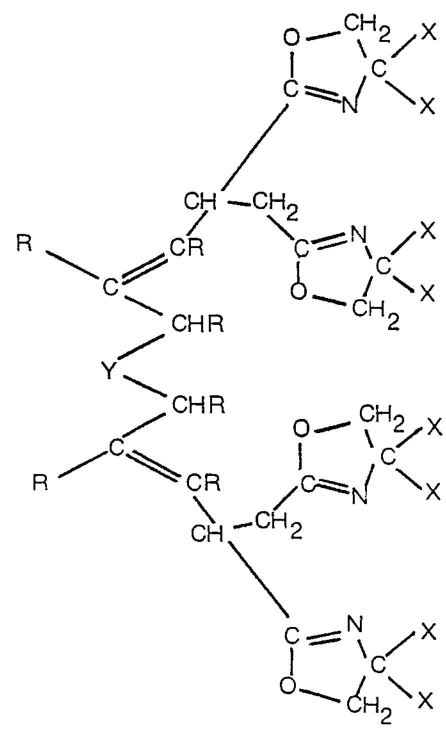

dans laquelle R est l'hydrogène ou est dérivé d'un polymère oléfinique dont le poids moléculaire a une moyenne en nombre de 500 à 140 000, X est un groupe alkyle ou hydroxyalkyle et l'un au moins des substituants X est un groupe hydroxyalkyle de formule $—(CH_2)_nOH$, dans laquelle n a une valeur de 1 à 3 et Y représente —S— ou —S—S—.

12. Composé suivant la revendication 11, dans lequel les deux substituants X représentent un groupe hydroxyalkyle de formule $—(CH_2)_nOH$ et n est égal à 1.

13. La dithio-bis-(polyisobuténył-bis-(5,5-bis-méthylol-2-oxazoline)) suivant la revendication 11.

14. La thio-bis-(polyisobuténył-bis-(5,5-bis-méthylol-2-oxazoline)) suivant la revendication 11.

15. Composition comprenant une matière oléagineuse de la classe consistant en carburants et lubrifiants et au moins une quantité inhibitrice de corrosion d'un composé suivant la revendication 11.

16. Composition suivant la revendication 15, dans laquelle ladite matière oléagineuse est une huile minérale lubrifiante contenant 0,01 à 20% en poids dudit composé.

17. Composition suivant la revendication 15, dans laquelle ladite matière oléagineuse est une huile minérale lubrifiante contenant 20 à 90% en poids dudit composé.

18. Composition suivant la revendication 15, dans laquelle ladite matière oléagineuse est l'essence.

**Patentansprüche**

1. Verfahren zur Herstellung von Thio-bis-(Kohlenwasserstoff-bis-oxazolinen), dadurch gekennzeichnet, daß ein Molteil eines Acylierungsmittels mit der Formel

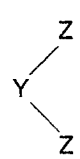

in der Y —S— oder —S—S— ist und Z der Rest eines Productes ist, das durch Umsetzung von Malein- oder Bernsteinsäure oder einem Ester oder einem Anhydrid davon mit einem Olefinpolymer mit einem zahlenmäßigen durchschnittlichen Molekulargewicht im Bereich von 500 bis 140.000 erhalten worden ist, mit 1 bis 4 Molteilen eines 2,2-disubstituierten-2-Amino-1-alkanols mit insgesamt 4 bis 8 Kohlenstoffatomen gemäß der Formel

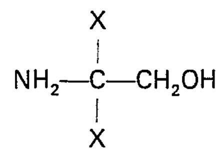

in der X eine Alkyl- oder Hydroxyalkylgruppe ist, wobei mindestens einer der X-Substituenten eine Hydroxyalkylgruppe der Struktur $-(CH_2)_nOH$ mit $n = 1$ bis 3 ist, umgesetzt wird, bis das Reaktionsprodukt bei der IR-Analyse eine maximale Absorption für Oxazolin zeigt.

2. Verfahren nach Anspruch 1, dadurch gekennzeichnet, daß das Acylierungsmittel Thio-bis-(polyisobutenylbernsteinsäureanhydrid) und das Alkanol tris(Hydroxymethyl)aminomethan ist.

3. Verfahren nach Anspruch 1 oder 2, dadurch gekennzeichnet, daß vier Molteile des Alkanols mit einem Molteil des Acylierungsmittels umgesetzt werden.

4. Verfahren nach Anspruch 1 oder 2, dadurch gekennzeichnet, daß 2 bis 3 Molteile des Alkanols umgesetzt werden mit einem Molteil des Acylierungsmittels und daß das Reaktionsprodukt dann mit Polyaminen und/oder Polyolen in einer ausreichenden Menge, um mit den verbliebenen Carboxylgruppen des Acylierungsmittels zu reagieren, umgesetzt wird.

5. Verfahren nach Anspruch 4, dadurch gekennzeichnet, daß das Alkanol tris(Hydroxymethyl)aminomethan und das Polyol Pentaerythrit ist.

6. Thio-bis-(Kohlenwasserstoff-bis-oxazolin) hergestellt durch Umsetzung des Reaktionsprodukts eines Olefinpolymeren mit einem zahlenmäßigen durchschnittlichen Molekulargewicht im Bereich von 500 bis 140.000 und Bernsteinsäureanhydrid, -säure oder -ester mit Schwefelmonochlorid oder Schwefeldichlorid unter Bildung eines chlorhaltigen Addukts, Dehydrochlorierung des Addukts durch Erwärmung unter Bildung eines Mono- oder Dithio-(hydrocarbyl-bernsteinsäureanhydrids oder -esters) und anschließende Umsetzung eines Molteils des Mono- oder Dithio-bis-Produkts mit 1 bis 4 Molteilen eines 2,2-disubstituierten-2-Admino-1-Alkanols mit insgesamt 4 is 8 Kohlenstoffatomen gemäß der Formel

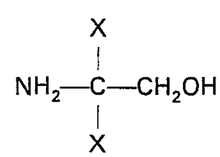

in der X eine Alkyl- oder Hydroxyalkylgruppe ist, wobei mindestens einer der X-Substituenten eine Hydroxyalkylgruppe der Struktur $(CH_2)_nOH$ mit $n = 1$ bis 3 ist, bis das Reaktionsprodukt bei der IR-Analyse eine maximale Adsorption für Oxazolin zeigt.

7. Thio-bis-(Kohlenwasserstoff-bis-Oxazolin) nach Anspruch 6, dadurch gekennzeichnet, daß das Alkanol tris(Hydroxymethyl)- aminomethan ist.

8. Thio-bis-(Kohlenwasserstoff-bis-oxazolin) nach Anspruch 6 oder 7, dadurch gekennzeichnet, daß 4 Molteile des Alkanols mit einem Molteil des Mono- oder Dithio-bis-Produkts umgesetzt worden sind.

9. Thio-bis-(Kohlenwasserstoff-bis-oxazolin) nach den Ansprüchen 6 bis 8, dadurch gekennzeichnet, daß das Olefinpolymer Polyisobutylen ist.

10. Thio-bis-(Kohlenwasserstoff-bis-oxazolin) nach Anspruch 6 oder 7, dadurch gekennzeichnet, daß das Alkanol in einer Menge von 1 bis 3 Molteilen verwendet und das Reaktionsprodukt anschließend mit Polyaminen und/oder Polyolen in einer ausreichenden Menge, um mit den verbliebenen Carboxylgruppen des Acylierungsmittels zu reagieren, umgesetzt worden ist.

11. Thio-bis-(Kohlenwasserstoff-bis-oxazolin) nach Anspruch 6 gekennzeichnet durch die Formel

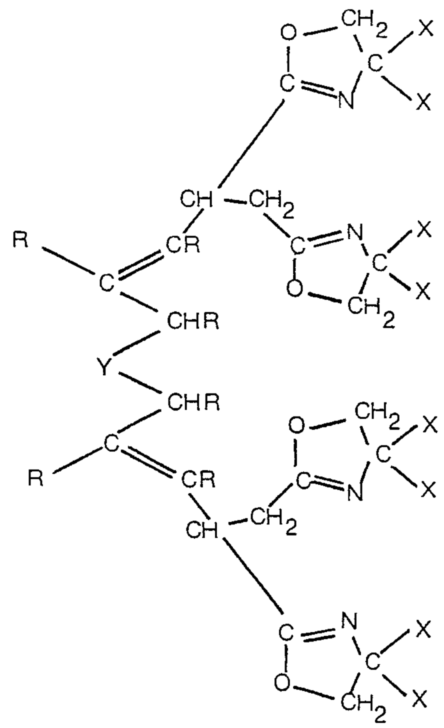

in der R Wasserstoff ist oder sich von einem Olefinpolymer mit einem zahlenmäßigen durchschnittlichen Molekulargewicht von 500 bis 140.000 ableitet, X eine Alkyl- oder Hydroxyalkylgruppe ist, wobei mindestens einer der X-Substituenten eine Hydroxyalkylgruppe mit der Struktur $—(CH_2)_nOH$ mit n = 1 bis 3 ist, und Y —S oder —S—S ist.

12. Verbindung nach Anspruch 11, dadurch gekennzeichnet, daß beide X-Substituenten eine Hydroxyalkylgruppe mit Struktur $—(CH_2)_nOH$ sind und n = 1 ist.

13. Dithio-bis-(polyisobutenyl-bis-(5,5,-bis-methylol-2-oxazolin)) nach Anspruch 11.

14. Thio-bis-(polyisobutenyl-bis-(5,5,-bis-methylol-2-oxazolin)) nach Anspruch 11.

15. Zusammensetzung gekennzeichnet durch ein öliges Material aus der Klasse bestehend aus Brennstoffen und Schmiermitteln und mindestens ein korrosioninhibierende Menge einer Verbindung gemäß Anspruch 11.

16. Zusammensetzung nach Anspruch 15, dadurch gekennzeichnet, daß das ölige Material ein Schmiermineralöl ist, das 0,01 bis 20 Gew.% der Verbindung enthält.

17. Zusammensetzung nach Anspruch 15, dadurch gekennzeichnet, daß das ölige Material ein Schmiermineralöl ist, das 20 bis 90 Gew.% der Verbindung enthält.

18. Zusammensetzung nach Anspruch 15, dadurch gekennzeichnet, daß das ölige Material Benzin ist.